# EUROPEAN PATENT APPLICATION

(11) **EP 4 477 226 A1**
(43) Date of publication of application: **18.12.2024**
(21) Application number: 23720706.3
(22) Date of filing: 09.02.2023
(51) Int. Cl.: A61K 35/74, A61K 39/04, A61P 35/00, A61K 39/39

(54) **COMPOSITIONS COMPRISING RECOMBINANT STRAINS OF MYCOBACTERIUM, USES THEREOF AND METHODS FOR THE PREVENTION AND/OR TREATMENT OF CANCER**

(30) Priority: 11.02.2022 BR 102022002660
(71) Applicant: INSTITUTO BUTANTAN, 05503900 São Paulo - SP (BR)
(72) Inventor: LEITE, Luciana Cezar de Cerqueira, 05083-030 São Paulo - SP (BR); MORENO, Ana Carolina Ramos, 05633-010 São Paulo - SP (BR); SOTO, Dunia Del Carmen Rodriguez, 01230-010 São Paulo - SP (BR); TRENTINI, Monalisa Martins, 05586-001 São Paulo - SP (BR)
(74) Representative: AWA Sweden AB
(86) International application number: PCT/BR2023/050044
(87) International publication number: WO 2023/150848

(57) **Abstract**

The present invention refers to the use of recombinant *Mycobacterium* strains encoding the A subunit of the heat-labile LT toxin from mutated *Escherichia coli,* in the manufacture of an immunobiological composition for the prevention and / or treatment of cancer in animals. Preferably, the mutation is at position 63 from serine to lysine. The present invention also provides immunobiological compositions comprising said strains for use in the prevention and / or treatment of cancer. At last, the present invention refers to methods for the prevention and / or treatment of cancer, comprising the administration of said strains to a subject in need thereof. In particular, said cancer is bladder cancer.

## Description

### TECHNICAL FIELD OF THE CLAIMED SUBJECT-MATTER

The present patent application refers to immunobiological compositions comprising recombinant molecules for the treatment of cancer, as well as methods and related uses.

### BACKGROUND OF THE SUBJECT-MATTER CLAIMED

### Cancer Immunotherapy

Although the current treatment for cancer relies mainly on the use of surgery, irradiation and chemotherapy, the evolution in the understanding of the biology of malignant transformation and the differences in the control of normal cell and cancer cell proliferation has provided the discovery of new potential targets for the treatment of cancer by immunotherapy.

The immune response is characterized as Th1 or Th2 according to the pattern of cytokines produced by lymphocytes T CD4+ and CD8+. The Th1 response is linked to the cellular immunity, which protects the organism against specific pathogens, besides providing antitumor action, while Th2 favors the humoral response, which is mediated by the action of antibodies. Their responses interact with each other, with inhibitory and / or regulatory effects, and in subjects affected by neoplasms, a negative modulation of the cellular response is observed (Van Kempen LC, et al. Differentiation research in biological diversity 2002; 70: 610 - 623).

The knowledge acquired in the field of immunology, especially regarding innate immunity, has provided important evidence of the connection between inflammation and cancer, completely transforming the understanding of neoplastic pathophysiology. Today, immunotherapy is used in an attempt to cure or modify tumor growth by intensifying the patient's immune response against tumor antigens (Mantovani, et al. Nature 2008; 454: 436 - 444) .

### Use of bacillus Calmette-Guérin (BCG) in cancer therapy

At the beginning of the last century, Albert Calmette and Camille Guérin attenuated a virulent strain of *Mycobacterium bovis,* curretnly known as bacillus Calmette-Guérin (BCG) and successfully used as tuberculosis vaccine.

In 1976, Morales, Eidinger and Bruce were the first to report the successful treatment of superficial bladder cancer with bacillus Calmette-Guérin (BCG) (Morales A, Eidinger D, Bruce AW. The Journal of Urology 1976; 116: 180 - 183). Since then, this immunotherapy in the form of intravesical BCG infusion has been used to treat and prevent recurrence of superficial bladder tumors. BCG has also shown efficacy against other types of tumors, such as melanoma, lung cancer, and leukemia (Patent US 5,712,123).

Although the mechanism of the antitumor effect of BCG is not completely defined, there is a general consensus that intravesical application of BCG causes a nonspecific inflammatory reaction of the immune system. Thus, it leads to T cell activation and infiltration, inducing predominantly the production of cytokines related to the Th1 response, the major responsible for the antitumor effect. There is also stimulation of the Th2 response, such as the release of IL-4, IL-5, IL6 and IL-10 cytokines, which may be related to the local and systemic side effects of intravesical immunotherapy with BCG (Luo Y. et al. Clinical and experimental immunology 2006; 146: 181 - 188).

About 90 % of bladder tumors are transitional cell carcinomas (TCC) that arise in the inner lining of the bladder called the urothelium. Of these, about 75 % of the cases have recurrence, where 45 % lead to progression to invasive disease (Sylvester RJ. et al. The Journal of Urology 2005; 174: 86 - 91).

The clinical therapy of bladder cancer is based mainly on antineoplastic phenomena resulting from the stimulation of immune responses of cellular and tumor type. Despite advances in the search for new therapies, such as the use of Epirubicin, Doxorubicin, Gencitabine, and Mitomycin C, intravesical application of BCG continues to show the greatest efficacy in preventing progression recurrence in both urothelial carcinoma and carcinoma *in situ* (CIS) of the bladder. (Bohle, A. et al. The Journal of Urology 2003; 169: 90 - 95).

The great advance in the development of therapies based on modulation of the immune response was the result of *in vivo* research. The animal models provided studies of the pathophysiology, immunology of bladder tumor and even evaluation of side effects of new therapies. The syngeneic orthotopic model uses MB-49 cells from C57BL/6. MB-49 cells are known to induce IL-10 and IL-4 production that direct the immune system towards Th2 type response, thus hindering a more effective antitumor reaction. This important characteristic is also found in human bladder tumors; therefore, the animal model with MB-49 cells presents an immune response similar to the human one, favoring comparative studies (Gunther, J. H. et al. Cancer Res 1999; 59: 2834 - 2837).

### Recombinant BCG

BCG is the most widely used vaccine in the world because it has a long-lasting immune response and a very low frequency of serious adverse effects, which makes it an excellent candidate as a vehicle for the presentation of heterologous antigens generating recombinant BCG-based vaccines (Patent US 6,673,353).

The immunotherapeutic mechanism of BCG was better understood with the identification of the genes responsible for the antitumor effect, enabling the production of recombinant organisms capable of stimulating a more intense Th1 response, aiming at a reduction of the dose to be employed and, consequently, fewer side effects.

In this sense, the expression of immunogenic domains of bacteria, viruses and parasites has been successfully used in BCG, generating recombinant strains (rBCG) potentially useful in cancer immunotherapy. For example, the feasibility of using rBCG as a strategy for the expression of cytokines, pharmacological agents, and antitumor agents is already known in the state of the art, providing the basis for a new strategy to treat certain types of cancer (Patent US 5,776,465). The use of this strategy gives rBCG the ability to express specific proteins or polypeptides that act as cell growth inhibitors or as cytotoxic substances for cancer cells (e.g., interferon α or β, interleukins 1-7 or tumor necrosis factor α or β).

Furthermore, it has been shown that rBCG vaccines are able to promote both humoral and cellular immune response, being an option in the treatment of certain types of cancer. The possibility of using rBCG to express specific antitumor agents (Patent US 5,776,465) is also already known as a possible alternative in cancer treatment.

### Heat-labile LT toxin from Escherichia coli and its immunomodulatory properties

Adjuvant properties have been attributed to several bacterial toxins. For example, it is widely known that the tetanic (TT), diphtheric (DT), cholera (CT), and the heat-labile (LT) toxin of *Escherichia coli* (*E. coli*), act as adjuvants that direct the immune response toward Th2 when co-administered with other antigens (Ryan, E.J. et *al.*

(2000) Modulation of innate and acquired immune responses by Escherichia coli heat-labile toxin: distinct pro- and antiinflammatory effects of the nontoxic AB complex and the enzyme activity. J Immunol. 165:5750-5759; Miyaji, E.N. et al. (2001) Induction of neutralizing antibodies against diphtheria toxin by priming with recombinant Mycobacterium bovis BCG expressing CRM197, a mutant diphtheria toxin. Infect Immun. 69: 869 - 874).

In particular, the toxin LT from *E. coli* is among the most potent adjuvants described to date (Lycke, N. et al. (1992) The adjuvant effect of Vibrio cholerae and Escherichia coli heat-labile enterotoxins is linked to their ADP-ribosyl transferase activity. Eur J Immunol. 22: 2277-2281; Pizza, M. et al. (2001) Mucosal vaccines: non-toxic derivatives of LT and CT as mucosal adjuvants. Vaccine, 19: 2534 - 2541).

The toxin LT from *E. coli* consists of a single molecule of A subunit (LTA, 27kDa), with ADP ribosyl transferase activity, linked to a pentamer of the B subunit (LTB, 11,6 kDa each) which binds to the ganglioside receptor GM1 of mammalian cells. The B subunit of LT is a potent signaling molecule capable of modulating the immune response. The immunostimulatory effect of LTB seems to be related to its ability to increase antigen presentation via major histocompatibility complex class I (MHC-I) and class II (MHC-II), among other factors. The adjuvant effect of LTB, on the other hand, has been directly related to GM1 binding activity, through the activation of B cells and CD4+ T cells by the interaction of B subunit pentamers and the GM1 receptors of these cells. In addition, LTB increases antigen presentation by activation of dendritic cells (DCs) and other antigen-presenting cells (APCs). The binding of LTB to GM1 ganglioside allows the toxic A subunit to enter the cell (Spangler, B.D. (1992) Structure and function of cholera toxin and the related Escherichia coli heat-labile enterotoxin. Microbiol Rev. 56: 622 - 647).

The use of LT as an adjuvant is not recommended because of the toxicity of subunit A. Subunit B, on the other hand, does not present this toxicity and has been more frequently used as an adjuvant (de Haan, L., Verweij, W.R., Feil, I.K., Holtrop, M., Hol, W.G., Agsteribbe, E. and Wilschut, J. (1998) Role of GM1 binding in the mucosal immunogenicity and adjuvant activity of the Escherichia coli heat-labile enterotoxin and its B subunit. Immunology, 94: 424 - 430).

The immunomodulatory properties leading to increased immunogenicity and protective efficacy of LT have been widely studied. Although the mechanisms leading to this effect are not well characterized, some aspects such as increased inflammatory cytokines and chemokine production, as well as transient recruitment of immune effector cells to the site of inflammation, have been established as important factors. LT can also influence dendritic cell maturation, antigen presentation and T cell activation and promote the induction of antigen-specific cytotoxic T cell response in animal model. The use of LT as an adjuvant commonly leads to a cytokine balance, involving the production of a response by cytokines with both Th1 and Th2 characteristics and some classes of antibodies in mice and humans.

This toxin is capable of activating an immune response against another antigen when presented simultaneously on the mucosal surface or by oral, intranasal or parenteral administration (Holmgren, J., Lycke, N. and Czerkinsky, C. (1993) Cholera toxin and cholera B subunit as oral-mucosal adjuvant and antigen vector systems. Vaccine, 11: 1179 - 1184).

As a consequence of these properties, LT has been employed extensively as an adjuvant in animal models. However, despite the immunomodulatory action of LT, it is highly toxic and inappropriate for clinical use in humans. Thus, to avoid the toxicity associated with the use of the native toxin while maintaining its adjuvant properties, different strategies have been used, including the isolation of the (non-toxic) B subunit and the construction of non-toxic LT mutants containing specific site-directed mutations. Through computational modeling studies of the structure of the LT protein, it was possible to identify some amino acids potentially involved in the enzymatic activity of this protein that could be studied by exchanging them through site-directed mutation (Magagnoli, C. et *al.,*

(1996) Infect Immun. 64: 5434 - 5438). Some of these mutants, such as LTR192G (substitution of arginine for glycine at position 192), have a mutation in a loop region that is protease sensitive, making this region insensitive to the action of proteases, a fundamental step for the activation of enzyme activity and consequently its toxicity. Other mutants have a mutation in the enzymatically active region of the subunit A, such as LTR72 (substitution of alanine for arginine at position 72), which retains only 1 % of ADP-ribosyl transferase activity.

Another important mutant is LTK63 (substitution of serine for lysine at position 63). This mutation eliminates the ADP-ribosyl transferase activity associated with toxicity, eliminating the same; however, it retains all other biological properties, including adjuvant properties of native LT (Pizza, M., et al., (2001) Mucosal vaccines: nontoxic derivatives of LT and CT as mucosal adjuvants. Vaccine, 19: 2534 - 2541).

LTK63 has been shown to act as a potent adjuvant when administered mucosally or parenterally. De Haan et *al.* (De Haan, L., Holtrop, M., Verweij, W.R., Agsteribbe, E. and Wilschut, J. (1999) Mucosal immunogenicity and adjuvant activity of the recombinant A subunit of the Escherichia coli heat-labile enterotoxin. Immunology, 97: 706 - 7013) suggest that adjuvants using non-toxic LTA in association with the LTB pentamer might be more potent than adjuvants using the B subunit alone, since the complex could stimulate the immune system more strongly than each molecule individually. Data pointing to an important function of the enzymatically inactive A subunit in inducing an immune response as well as in immunomodulatory activities, such as effects on antigen processing and presentation, support this view.

### Expression of LT and its variants

Recombinant LTB and LTK63 have typically been expressed in *E. coli.* However, other expression systems have been used. The LTB molecule has been expressed in *Mycobacterium bovis* BCG, *Lactobacillus casei, Saccharomyces cerevisiae, Pichia pastoris,* as well as plants including *Oryza sativa* (rice), *Lactuca sativa* (lettuce) and *Peperomia pellucid.* LTK63 has also been expressed in tobacco and chloroplasts, as well as attenuated *Salmonella enterica* serovar Typhimurium (da Hora, V.P. et al. (2011) Non-toxic derivatives of LT as potent adjuvants. Vaccine, 29: 1538 - 1544) .

The goal of all this work, however, was to use the LT molecule or one of its subunits or variants to produce vaccines against *E. coli,* not to treat cancer.

Furthermore, with regard to cancer treatment, Brazilian patent application PI 0904468-0 discloses an immunotherapeutic comprising a recombinant *Mycobacterium bovis* (BCG) strain encoding the subunit 1 of the pertussis toxin (S1PT) of *Bordetella pertussis.* This immunotherapy showed good results for the treatment of bladder cancer. For example, there was retardation of tumor growth with significant reduction of tumor volume; increased efficiency of splenocytes in destroying tumor cells; and increased survival curve in the group of mice with bladder tumor treated with rBCG-S1PT compared to the control group. However, there is still a need for other rBCG alternatives for use in cancer immunotherapy.

The rBCG involved in the present invention is completely distinct from any other used for the same purpose, since the recombinant *Mycobacterium* strains described herein encode the A subunit of the heat-labile LT toxin from mutated *Escherichia coli* referred to in this patent application is described in patent application BR 11 2014 020297 4, national phase of international patent application PCT/BR2013/000049, filed on February 18, 2013.

### BRIEF DESCRIPTION OF THE SUBJECT-MATTER CLAIMED

The present invention refers to the use of recombinant *Mycobacterium* strains encoding the A subunit of the heat-labile LT toxin from mutated *Escherichia coli,* in the manufacture of immunobiological compositions for the prevention and / or treatment of cancer.

The present invention also refers to the use of recombinant *Mycobacterium* strains encoding the A subunit of the heat-labile LT toxin from mutated *Escherichia coli* at position 63, in the manufacture of immunobiological compositions for prevention and / or treatment of cancer. Preferably, the mutation at position 63 is a serine to lysine substitution.

The present invention also provides immunobiological compositions comprising said strains for use in the prevention and / or treatment of cancer.

At last, the present invention refers to methods and uses for the preparation of immunobiological compositions and for prevention and / or treatment of cancer comprising administering said strains, or immunobiological compositions comprising said strains, to a subject in need thereof.

### OBJECTIVES

The strains described throughout this document had the unexpected technical effect of provoking a more intense antitumor immune response when compared to conventional BCG, with respect to the prevention and / or treatment of cancer.

Thus, it is an objective of the present invention to describe the use of recombinant *Mycobacterium* strains in the manufacture of an immunobiological composition to treat and / or prevent cancer, in which the recombinant *Mycobacterium* strains encoding the A subunit of the heat-labile LT toxin from the mutated *Escherichia coli.*

In particular, it is an objective of the present invention the use of *Mycobacterium* strains, preferably recombinant *Mycobacterium bovis, Bacillus Calmette Guerin* (BCG), encoding the A subunit of the heat-labile LT toxin from *Escherichia coli,* mutated at position 63 from serine to lysine, rBCG-LTAK63, in the manufacture of an immunobiological composition for the treatment and / or prevention of cancer.

It is also an objective of the present invention to provide immunobiological compositions comprising said strains for use in the prevention or treatment of cancer.

The present invention further aims methods for the prevention and / or treatment of cancer comprising the administration of said strains to the subject in need of the same.

### DEFINITIONS

In the scope of this patent application, abbreviations are used several times, the definitions of which are summarized below:
- BCG refers to attenuated *Mycobacterium bovis,* bacillus Calmette-Guérin;
- LTAK63 refers to A subunit of the heat-labile toxin from *Escherichia coli,* modified by site-directed mutagenesis; and
- rBCG-LTAK63 refers to recombinant BCG expressing LTAK63.

### DESCRIPTION OF THE FIGURES

The following figures are part of the present application and are included herein in order to illustrate certain aspects of the invention.
Figure 1 shows an agarose gel containing the PCR products confirming the presence of the LTAK63 gene fragment in recombinant BCG (rBCG-LTAK63). PCR products were amplified from plasmid DNA extracted from the rBCG-LTAK63 construct using primer oligonucleotides specific for amplifying the LTA subunit. 1Kb plus, molecular weight; well 1, full LTA fragment (~700 pb).
Figure 2 shows the characterization of LTAK63 (~ 31.0 kDa) expression in recombinant BCG. Soluble protein extract (~ 10 µg) of rBCG transfected with pLNIP- LTAK63 (A), or empty BCG as a negative control (B) were used in this immunoassay. A serum (polyclonal) made from rabbit (anti-LT 1:1000) was used.
Figure 3 shows the average bladder weights in the groups that received MB49 tumor cell implants and were treated with the immunobiological strains / compositions described herein.
Figure 4 shows the survival of BCG-, rBCG-LTAK63- and control-treated mice after intravesical implantation of MB49 tumor cells.
Figure 5 shows the activation of CD8+ T lymphocytes, NK cells and macrophages present in the bladder / tumor of mice analyzed thirty days after intravesical implantation of MB49 tumor cells and four cycles of treatment with saline (control), BCG or rBCG-LTAK63.

### DETAILED DESCRIPTION OF THE SUBJECT-MATTER CLAIMED

### Description of the strains

In an embodiment, the present invention refers to the use of recombinant *Mycobacterium* strains encoding the A subunit of the heat-labile LT toxin from mutated *Escherichia coli.* More preferably, the *Mycobacterium* strains encoding the A subunit of the heat-labile LT toxin from *Escherichia coli* are mutated at position 63 from serine to lysine and are called rBCG-LTAK63.

According to this embodiment, said mutations allow the A subunit of the heat-labile LT toxin from *Escherichia coli* encoded by *Mycobacterium* maintain the adjuvant properties of native LT, but lose the ADP-ribosyl transferase activity associated with toxicity, thus avoiding toxicity problems in the case of humans or other animals.

The strains of the present invention are obtained from any strain of the genus *Mycobacterium* as carrier for presentation of one or more antigens of distinct organisms, obtained and cloned into a mycobacterial expression vector and inserted by genetic manipulation.

Preferably the recombinant *Mycobacterium* strains used in the present invention comprise strains of the MTB complex, *Mycobacterium tuberculosis, Mycobacterium bovis, Mycobacterium microft* and *Mycobacterium africanum* or of fast-growing mycobacteria, *Mycobacterium smegmatis, M. aurum, M. vaccae, etc.*

More preferably, the recombinant *Mycobacterium* strains of the present invention comprise bacillus Calmette Guerin (BCG) *Mycobacterium bovis* strains.

### Use of the strains

as can be seen in the Examples below, said strains have the unexpected technical effect of provoking a more intense anti-tumor immune response when compared to traditional BCG or other rBCG.

The addition of an immunogenic domain of the non-toxic LTAK63 subunit of the heat-labile toxin from *E. coli,* which is known to have no link to any type of cancer, in a recombinant mycobacterium, in particular rBCG, causes an increase in the anti-tumor immune response of rBCG.

In particular, the strains of the present invention promote a decrease in tumor volume in bladder tumor animal models.

The strains of the present invention, by combining mycobacteria, in particular BCG, and toxins derived from other pathogens, preferably *Escherichia coli,* to generate a recombinant mycobacterium strain, can be used to manufacture immunobiological compositions for the prevention or treatment of cancer, where such compositions are more potent and effective than those comprising conventional BCG, or other rBCG, for the treatment of cancer, particularly but not limited to bladder cancer.

Considering the properties of the recombinant *Mycobacterium* strains described throughout this document, the present invention describes the use of one or more of these strains in the manufacture of an immunobiological composition for the prevention and / or treatment of cancer in animals, more particularly humans.

Another aspect of the present invention is to provide immunobiological compositions comprising these strains for use in prevention and / or treatment of cancer in animals.

Such immunobiological compositions can comprise one or more of a pharmaceutically acceptable vehicle, excipient, diluent or solvent, i.e., materials that can comprise: non-toxic solid, inert, semi-solid liquid excipient, diluent, auxiliary formulation of any kind, or simply a sterile aqueous medium, such as saline solution. Other examples of excipients: bactericides, bacteriostatics, antioxidants, preservatives, buffers, stabilizers, pH adjusters, osmolarity adjusters, antifoaming agents, and surfactants; and residues from antigen inactivation or fractionation agents, among others.

It constitutes yet another aspect of the present invention methods for the prevention or treatment of cancer in animals, more particularly humans, by administering said strains or immunobiological compositions to the subject in need thereof.

In particular, said cancer is bladder cancer.

Thus, the recombinant *Mycobacterium* strain disclosed in this patent application, encoding the A subunit of the heat-labile LT toxin from mutated *Escherichia coli* can be used in the manufacture of an immunobiological composition for the treatment and / or prevention of cancer, particularly bladder cancer.

### EXAMPLES

In order to allow a better understanding of the present invention and to clearly demonstrate the technical advances obtained, the results of the different tests conducted are presented below as examples.

In Example 1, the obtaining of the strains and their use in the preparation of immunobiological compositions is described. In the other Examples (2 to 3), the properties and use of the strains or immunobiological composition in the treatment of bladder cancer are illustrated. These Examples are presented for illustrative purposes only and should in no way be considered as limiting the scope and range of the present invention.

### EXAMPLE 1: Obtaining the strains and immunobiological compositions of rBCG-LTAK63

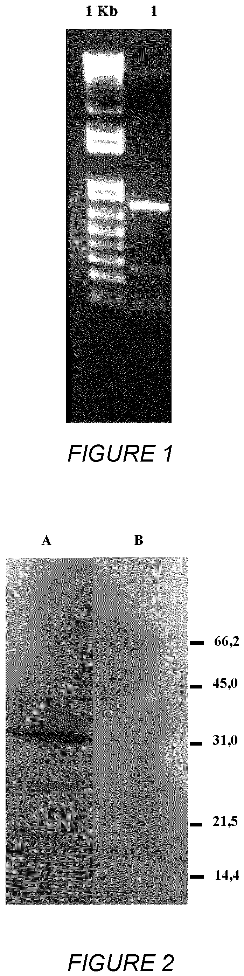

**a) Preparation of stock lot of competent BCG:** for the preparation of competent BCG stocks, one or more BCG colonies were grown in Middlebrook 7H9 liquid medium plus Tween-80 and supplemented with 10 % albumin-dextrose-catalase (MB7H9 / Tw / ADC) until exponential phase. Composition of Middlebrook 7H9 medium according to the manufacturer (Difco-BD): Ammonium sulfate, L-Glutamic acid, Sodium citrate, Pyridoxine, Biotin, Sodium di-phosphate, Potassium monophosphate, Ferric ammonium citrate, Magnesium sulfate, Calcium chloride, Zinc sulfate, and Copper sulfate. The culture was then sedimented by centrifugation at 4000 rpm and washed twice with 10 % glycerol at 40 °C, finally re-suspended in 5 % of the original volume with 10 % glycerol and stored at -70 °C until transformed by electroporation with plasmid pNL12-LTAK63.
**b) Construction of the LTAK63 gene expression vector in recombinant BCG herein named pLN12-LTAK63:** the recombinant BCG expression vector of the LTAK63 gene was constructed using conventional molecular biology methods. The LTAK63 gene was amplified using PCR from the LTK63 gene using the following primer oligonucleotides: N-terminal [SEQ ID NO: 1] (5'-**TAGGGATCCAATGGCGACAGATTATACCGTTG**-3'), which contains the BamH I restriction site (underlined) and C-terminal [SEQ ID NO: 2] (5'-**TAGGGTACCTAATTCATCCCGAATTCTGTTATA**-3'), containing the Kpn I restriction site (underlined). The PCR product corresponding to the LTAK63 gene was generated using the following PCR conditions: 94 °C for 4 min; 25 cycles of 94 °C for 1 min, 50 °C for 1 min, and 72 °C for 30 s; and 4 °C final. This PCR product was then digested with the BamH I and Kpn I restriction enzymes and then cloned into the pMIP12 expression vector, which was also previously digested with the same BamH I and Kpn I enzymes, thus generating the expression vector named pLNIP-LTAK63. The gene sequence of plasmid pLN12-LTAK63 comprises SEQ ID NO: 3.
**c) Obtaining the recombinant BCG strain expressing the A subunit LTAK63, rBCG-LTAK63:** For the preparation of rBCG-LTAK63, a stock aliquot of 50 - 200 µl competent BCG was mixed with 0.1 - 1 µg of pLN12-LTAK63 in 2 mm electroporation cuvettes and subjected to 2.5 kV, 25 µF, 1000 Q pulsations in a pulsation electroporator (GenePulser, BioRad, Hemel Hempstead, UK). After electroporation, the contents of the cuvettes were recovered in 2 mL of medium (MB7H9 / Tw / ADC) without antibiotic and incubated at 37 °C for 20 h before being seeded on Middlebrook 7H10 solid medium (Difco) supplemented with 10 % oleic acid - albumin - dextrose - catalase (MB7H10 / OADC) plus kanamycin (20 µg / mL) for selection of transformants. Approximate composition of Middlebrook 7H10 solid medium according to the manufacturer (Difco-BD): Ammonium Sulfate, Potassium Monophosphate, Potassium Bi-Phosphate, Sodium Citrate, Magnesium Sulfate, Calcium Chloride, Zinc Sulfate, Copper Sulfate, L-Glutamic Acid, Ferric Ammonium Citrate, Pyridoxine Hydrochloride, Biotin, Malachite Green and Agar. Selected rBCG colonies were then transferred to 5 mL of liquid MB7H9 / Tw / ADC culture medium with kanamycin (20 µg / mL) .
**d) Preparation of rBCG-LTAK63 batches:** clones of the strains were then expanded into 50 mL of liquid MB7H9 / Tw / ADC medium or any medium described for cultivation of mycobacteria plus kanamycin (20 µg / mL). After 2 - 3 weeks, when the culture reaches OD₆₀₀ 0.6 - 0.8, the samples were centrifuged, washed twice with distilled H₂O, resuspended in 1 mL of 10 % glycerol and aliquoted in 50 µl volume and then stored at -80 °C for later use in immunization assays.
**e) Evaluation of rBCG-LTAK63 batches:** the viability of the rBCG-LTAK63 batches was assessed by counting the number of colony forming units (CFU). The number of CFU was determined as follows: one or more aliquots of the batch frozen at -80 °C were thawed and several successive dilutions (1×10², 1×10⁴, 1×10⁵ and 1×10⁶) were performed. Then the 1×10⁵ and 1×10⁶ dilutions were plated on MB7H10 / OADC medium plus kanamycin (20 µg / mL) and incubated at 37 °C. After 3 - 4 weeks, the number of colonies on the plate was counted.
**f) Characterization of LTAK63 expression in recombinant BCG:** To verify LTAK63 gene expression, a Western blot immunoassay using a polyclonal anti-LT serum was used as follows: one or more stock aliquots of rBCG-LTAK63 were sonicated for 1.5 min on ice at a constant amplitude corresponding to half the maximum value (Soniprep 150 MSE, UK) and centrifuged to precipitate solids. The supernatants were recovered and the protein concentration dosed using the BIO-RAD Protein Assay kit (Bio-Rad), using bovine albumin as a standard. Samples containing 10 µg of total proteins were applied to a 10 % polyacrylamide gel in the presence of sodium dodecyl sulfate (10 % SDS-PAGE). Electrophoresis was performed at room temperature at 120 V until the dye reached the end of the gel. After electrophoresis, proteins were transferred to PDVF membrane with 0.45 µm pore size (GE Healthcare) using a semi-dry transfer system. The gel containing LT, as a positive control, was sandwiched under the PDVF membrane, dipped in transfer buffer (0.25 M Tris, pH 8.3, 0.129 M glycine and 20 % methanol) and placed between five filter sheets also soaked in the same buffer. The assembly was placed between two plates and subjected to a current of 120 mA for 1.5 h at room temperature. At the end of the electro transfer, the membrane was removed from the system and incubated in blocking solution (PBS containing 5% milk - PBS-L) at 4oC overnight. At the end of blocking, the membrane was incubated at room temperature for 2 h with anti-LT serum diluted in PBS-L (1:1000 dilution). After this incubation period the PVDF membrane was washed three times, under gentle agitation, with 0.1 % PBS / Tween20 (PBS-T) at 10 min intervals. The membrane was then incubated for 2 h under the same conditions in PBS-L containing HRP-conjugated anti-IgG antibody (Sigma, Chem Co, St. Luis) and again washed 3 times with PBS-T. Revealing was done by chemiluminescence using the ECL kit (Amersham) by exposure in a photo documentation apparatus (ImageQuant LAS4000 - GE) (Figure 2) .

### EXAMPLE 2: Treatment of bladder tumor animal models with immunotherapies.

**a) Bladder tumor animal model:** Adult females C57BL/6 mice were used (*Mus musculus,* Rodentia, Mammalia), weighing between 18 and 20 grams, 8 to 10 weeks old, originating from and maintained under standard conditions at the Central Animal Facility of the Medical School of the University of São Paulo.

We used the MB49 mouse urothelial bladder tumor cell line C57BL/6 (donated by Dr. Yi Lou - University of Iowa, USA), which was grown in RPMI 1640 medium supplemented with 10 % fetal bovine serum, 2 mmol / L L-glutamine, 50 U / mL penicillin, and 0.05 mg / mL streptomycin (Sigma Chemical Company, St. Louis, MO) in an incubator at 37 °C and 5 % CO₂. The MB49 cell suspension, stained with trypan blue, was quantified in a Neubauer chamber under the microscope, and only, suspensions with at least 90 % viable cells were used for tumor implantation into the bladder.

The implantation of the tumor cells followed the method described by Loskog, A. et al. (Lab Anim. 2005 Oct; 39(4): 384 - 93), with minor modifications described below. The mice were anesthetized by intramuscular (I.M.) injection of 100 µL of a solution composed of 1.0 mg of ketamine (Dopalen - Agribrands do Brasil - Paulinia / SP) and 0.1 mg of xylazine (Anasedam - Agribrands do Brasil - Paulinia / SP (GORSKA 2000). Then, the animals were submitted to transurethral catheterization, with a 24-gauge polyethylene catheter without needle. Then, the bladder epithelium was chemically injured with 0.1 mL of a 22 % ethanol solution for 15 minutes, followed by washing with 0.9 % saline solution (SS) and intravesical instillation of 0.1 mL of MB49 (5 × 10³ cells / mouse).

**b) Treatment with immunotherapies:** the animals were divided into three groups (n = 10):
**Control group:** animals treated with 0,1 mL of SF.
**BCG group:** animals treated with 0,1 mL of BCG suspension [1×10⁶ CFU / mouse suspended in saline solution (SF)];
**rBCG-LTAK63 group:** animals treated with 0,1 mL of rBCG-LTAK63 suspension (1×10⁶ CFU / mouse suspended in SF);

For the treatment, the animals were anesthetized again and had their urethra catheterized, as described. The treatments proposed for each group were carried out through one application per week, on days 1, 8, 15, 22, totaling 4 applications. On the 29th day, the mice were sacrificed, and the bladders were removed (cystectomy), weighed on precision analytical scales, frozen in liquid nitrogen and stored in a freezer -80 °C. Additionally, the bladders / tumors were processed with collagenase for further analysis of the activation of immune cells.

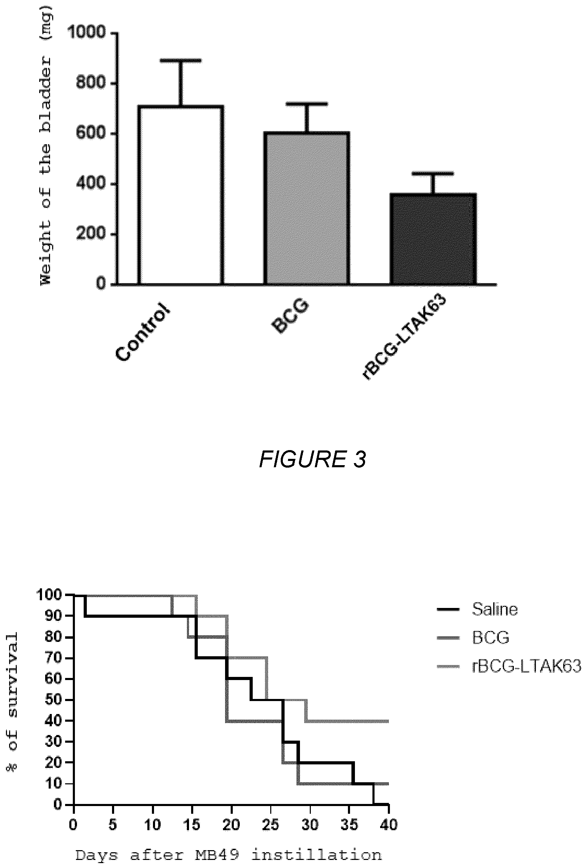

**c) Weight of the bladders: c) Weight of the** bladders: The evaluation of the weights of the bladder (mg), taken from the animals in this assay, showed statistically significant differences between the groups treated with BCG and rBCG-S1PT compared to the control group (SF) (Figure 3). One-way Analysis of Variance (p < 0.01) was used to analyze the difference in bladder weights between the various groups and the Student-Newman-Keuls test was used for pairwise analysis after the data were converted to the logarithmic scale. The mean weight (mg) of the control group was 700 ± 110 mg, the BCG group was 500 ± 87 mg, and the rBCG-LTAK63 group was 320 ± 50 mg. A p-value of < 0.05 was considered for statistically significant analysis. The values were obtained from three experiments performed independently. That is, the group that received saline solution had the highest bladder weights, and consequently the highest tumor volume. While the group treated with rBCG-LTAK63 showed the lowest bladder weights, which corresponds to a greater delay in tumor growth in the group treated with rBCG-LTAK63 compared to the control group.

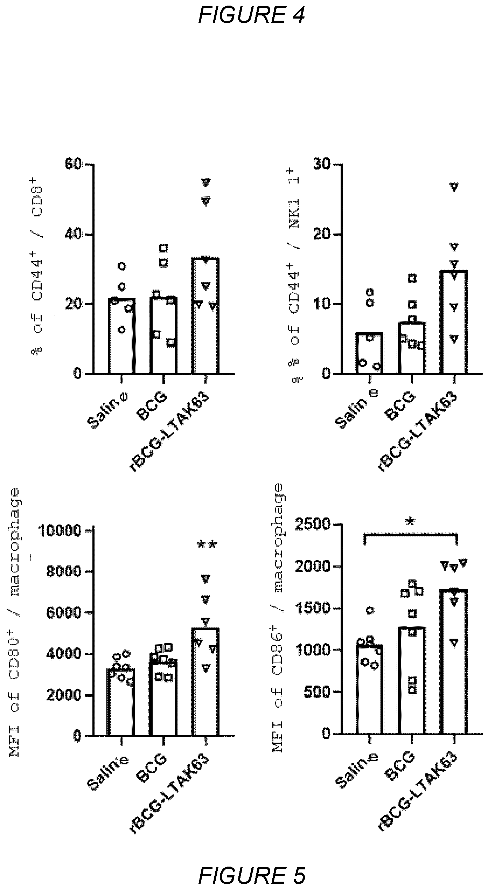

**c) Survival assay:** we proceeded as described in a) and b), but the animals were not sacrificed, but were followed for 40 days and, daily, the deaths of each group were counted. The individual survival curve of animals with bladder tumor showed a difference between the group treated with rBCG-LTAK63 and the others (Figure 4).

**d) Immunophenotyping assay:** the evaluation of the activation of immune cells present in the bladders / tumors, taken from the animals in this trial, showed statistically significant differences between the rBCG-S1PT treated groups compared to the control (SF) and BCG groups (Figure 5). One-way Analysis of Variance test was used (p < 0.01) to analyze the difference in immune cell activation between the various groups. A trend of increased activation of CD8+ T lymphocytes and NK cells was observed between the rBCG-LTAK63 group and the other groups. The activation of intravesical / intratumoral macrophages was statistically higher in the rBCG-LTAK63 group compared to the control and BCG groups. A p value of < 0.05 was considered statistically significant. Values were obtained from an experiment with n = 6.

## Claims

1. Use of a recombinant *Mycobacterium* strain encoding the A subunit of the heat-labile LT toxin from mutated *Escherichia coli* **characterized in that** it is for the manufacture of an immunobiological composition for the treatment and / or prevention of cancer.

2. Use, according to claim 1, **characterized in that** the strain encodes the A subunit of the heat-labile LT toxin from mutated *Escherichia coli.*

3. Use, according to any of the claims 1 or 2, **characterized by** the fact that the A subunit of the *Escherichia coli* heat-labile toxin LT is mutated in position 63.

4. Use, according to claim 3, **characterized in that** the mutation at position 63 is a substitution from serine to lysine.

5. Use, according to any one of claims 1 to 4, **characterized in that** the strain is selected from the group consisting of *Mycobacterium tuberculosis* strains, *Mycobacterium bovis* strains, *Mycobacterium microft* strains, *Mycobacterium africanum* strains, *or Mycobacterium smegmatis* strains, *Mycobacterium avium* strains, *Mycobacterium vaccae* strains, etc.

6. Use, according to claim 5, **characterized in that** the recombinant *Mycobacterium* strain is a *Mycobacterium bovis* Bacillus Calmette Guerin (BCG) strain.

7. Use, according to any one of the claims 1 to 6, **characterized in that** the cancer is bladder cancer.

8. Immunobiological composition for use in the prevention and / or treatment of cancer **characterized in that** it comprises one or more strains as defined in any one of claims 1 to 7 and one or more of a pharmaceutically acceptable vehicle, excipient, diluent, or solvent.

9. Method of prevention and / or treatment of cancer **characterized in that** it comprises the administration of one or more recombinant *Mycobacterium* strains as defined in any one of claims 1 to 7 or of an immunobiological composition as disclosed in claim 8, to a subject in need thereof.

10. Recombinant *Mycobacterium* strain encoding the A subunit of the heat-labile LT toxin from mutated *Escherichia coli,* as defined in any one of claims 1 to 9, **characterized in that** it is for use in the manufacture of an immunobiological composition for the treatment and / or prevention of cancer.
